# EUROPEAN PATENT SPECIFICATION

(11) **EP 2 917 189 B1**
(45) Date of publication and mention of the grant of the patent: **17.08.2016**
(21) Application number: 12787424.6
(22) Date of filing: 09.11.2012
(51) Int. Cl.: C07D 263/20, C07C 63/08

(54) **PROCESS FOR MAKING LINEZOLID**
VERFAHREN ZUR HERSTELLUNG VON LINEZOLID
PROCÉDÉ DE PRODUCTION DE LINÉZOLIDE

(43) Date of publication of application: 16.09.2015
(73) Proprietor: Synthon BV, 6545 CM Nijmegen (NL)
(72) Inventor: BARTL, Jiri, 67817 Blansko (CZ)
(74) Representative: Mendivil Gil, Maria Dolores
(86) International application number: PCT/EP2012/072265
(87) International publication number: WO 2014/071990

(56) References cited:
- EP-A1- 2 388 251
- WO-A2-2010/084514
- HOON-GYU PARK ET AL: "A New Method for the Oxazolidinone Key Intermediate of Linezolid and its Formal Synthesis", BULLETIN OF THE KOREAN CHEMICAL SOCIETY, vol. 33, no. 4, 20 April 2012 (2012-04-20) , pages 1389-1392, XP055070074, ISSN: 0253-2964, DOI: 10.5012/bkcs.2012.33.4.1389

## Description

### BACKGROUND OF THE INVENTION

The present invention relates to an improvement in the process for making the compound linezolid.

Linezolid is the generic name for N-(3-(3-fluoro-4-(morpholin-4-yl)phenyl)-2-oxooxazolidin-5(S)-ylmethyl)acetamide. It is represented by formula (I)

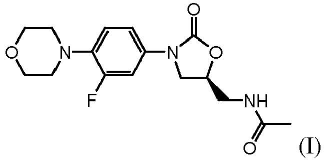

Linezolid is a pharmaceutically active compound useful as an antibacterial agent, e.g. for the treatment of diabetic food infections caused by Gram-positive bacteria.

The marketed pharmaceutical compositions sold, e.g., under brand name ZYVOX by Pfizer, are a sterile isotonic solution for an i.v. infusion, a tablet for oral administration, and an aqueous suspension for oral administration.

In the above-marketed compositions, linezolid is present as a free base.

Linezolid was first disclosed in WO 95/07271 (EP 0717738, US 5,688,792) of the Upjohn Company.

Various processes for making linezolid are known in the art. In particular, the important ones are these, the final step of which comprises acetylation of the amine of formula (II) 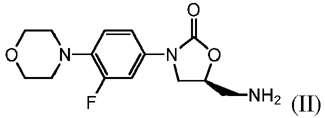 or an acid addition salt thereof with an acetylation agent, which typically is acetylhalide or acetic anhydride (see, e.g., WO 2005/099353), preferably in the presence of a base (WO2012/019862).

The above amine precursor of formula (II) may be made from various starting materials, e.g.:
a) By reduction of an azide compound of formula (III) by a suitable reductant (WO2006/091731, WO 95/07271, US 5837870, WO2009/063505, US 7291614) 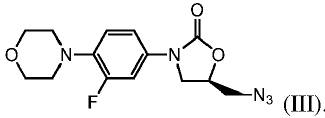
   The starting compound (III) may be made from the corresponding tosylate or chloride of general formula (VII) below (WO 2005/099353);
b) By decomposition of a phthalimide compound of formula (IV), e.g. by methylamine (WO95/07271) or by hydrazine (US 5837870) 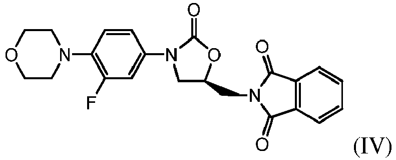
   The starting compound (IV) may be made from the same tosylate or chloride as sub a) (WO2005/099353) or by cyclization of the oxazolidine ring (WO 99/24393, WO2006/008754);
c) From a sulfonate compound of formula (V) 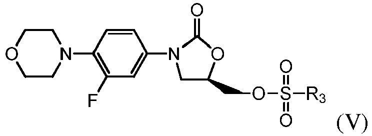 by treatment with ammonium hydroxide in isopropanol or THF (WO 95/07271) or by treatment with ammonia under enhanced pressure (WO 97/37980);
d) By reduction of an imine (VI) 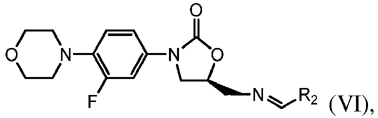 wherein R2 is a chlorophenyl, bromophenyl or 2,4,-dichlorophenyl moiety (WO 2007/116284);
e) By catalytic hydrogenation of a benzylamine intermediate (IIA) 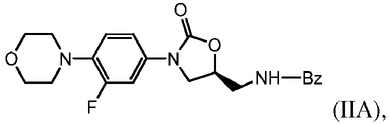 wherein Bz is an unsubstituted or substituted benzyl group ( WO 2010/084514).
   In an alternative disclosed in EP 2388251, the debenzylation hydrogenation reaction is carried out on a dibenzylamino- intermediate of formula (IIC) 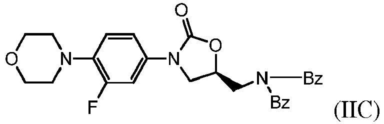 and the formed compound (II) is isolated either as a base or as an acetate salt;
f) By dealkylation of an alkyl/alkenyl- amine intermediate (IIB) 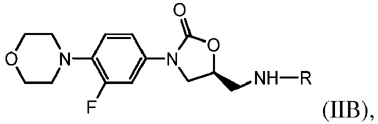 wherein R is a C4-C10 alkyl radical which is attached to the N-atom by a tertiary carbon atom or a C3-C10 alkenyl radical ( EP 2163547);
g) By reacting the compound of formula (VII) below with a metal salt of diformylamide of formula (VIII) 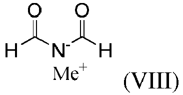 wherein Me⁺ is a sodium or potassium cation, and subjecting the reaction product comprising a mixture of compounds of formula (IXa) and (IXb), 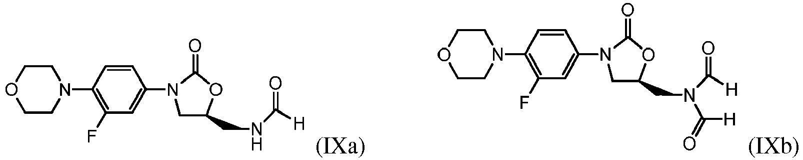 to a reaction with an acid, preferably with hydrochloric acid (WO 2012/019862).

Most of the preceding synthetic approaches are based on converting a starting material of general formula (VII) 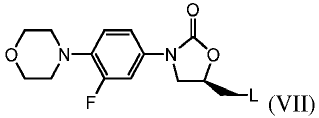 wherein L is a suitable leaving group, for instance a halogen or an alkyl-or aryl sulfonyloxy group, by reaction with a nitrogen nucleophile (an azide salt, phthalimide salt, benzyl/alkyl/alkenylamine, ammonia or ammonium hydroxide), followed, if necessary, by conversion of the formed reaction intermediate (e.g., compound (IIA), (IIB),(IIC), (III), (IV),(IXa) or (IXb) ) to the amino-compound of formula (II). The compound of formula (VII) is generally made from the compound of formula (X)

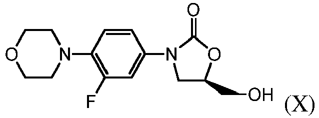

The amino-compound of formula (II) may be obtained in various solid state forms. The forms A, B and C were disclosed in US 2006/0258655. The compound (II) may be obtained also in the form of an acid addition salt. WO 2007/116284 disclosed a hydrochloride salt and WO2010/084514 disclosed a hydrochloride and a hydrobromide salt. WO 2010/081404 disclosed an acetate salt of the amine (II). In the specification it was mentioned that the acetate was obtained in high (chemical) purity, was easy to separate, and did not introduce any new impurities when converted into linezolid using acetic anhydride.

Apparently, making the starting amino-compound of formula (II) in a good yield and high chemical and/or optical purity is the key aspect of commercial success of any of the above synthetic routes yielding linezolid.

A suggestion for purifying the compound of formula (II) from side products has been presented in WO 2010/084514. The method is based on providing an acid addition salt, preferably hydrochloride salt, of the compound (II) by debenzylation of the compound (IIA) and then basifying the salt by a suitable base to get purer compound of formula (II). In an alternative disclosed in EP 2163547, a crude compound (II) was treated with HCl in a mixture of an organic solvent and water, the organic layer was removed and the aqueous solution of the hydrochloride salt of (II) was neutralized with sodium hydroxide. The aqueous mixture was extracted by an organic solvent and the purified compound (II) was isolated by evaporation.

Thus, while the compound (II) and a process for making it are known in the art, there still exists a need for improvement. It particular, it would be desirable to have a process for providing the amine-intermediate of linezolid of formula (II) in a good chemical and/or optical purity and in a solid form, which is well suitable for conversion into linezolid.

### SUMMARY OF THE INVENTION

The present invention relates to the discovery of an improvement in making linezolid, which provides for a product of high chemical and enantiomeric purity. In particular, the invention deals with an improvement in purification of the key intermediate of formula (II).

In a first aspect, the invention provides a benzoate salt of N-(3-(3-fluoro-4-(morpholin-4-yl)phenyl)-2-oxooxazolidin-5(S)-ylmethyl)amine of formula (II-D).

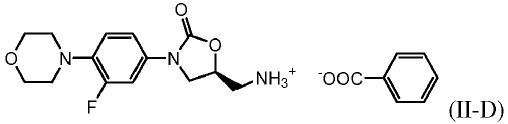

In a particular aspect, the benzoate salt is provided in a solid, preferably crystalline, form.

In a second aspect, the invention provides for a process for making the benzoate salt of formula (II-D), comprising reacting the compound of formula (II) with benzoic acid in a solvent. In a particular aspect, the benzoate salt is provided in a solid, preferably crystalline, form.

In a third aspect, the invention provides for a process for improving the purity, particularly the optical purity, of the compound of formula (II) comprising converting the compound of formula (II) into the benzoate salt of formula (II-D) in a solvent, preferably an alcoholic solvent, precipitating and isolating the benzoate salt and optionally, converting the isolated benzoate salt into the compound of formula (II).

In a fourth aspect, the invention provides for a process for making the benzoate salt of formula (II-D), characterised in that the leaving group L in the precursor of formula (VII) 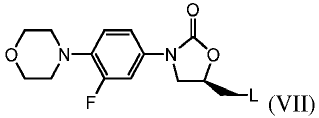 wherein L is for instance a halo- group or an alkyl- or aryl sulfonyloxy group, is converted into an amino-group in the presence of, or followed by a treatment with, benzoic acid. In a particular aspect, the group L is converted into an amino-group by reaction of the compound of formula (VII) with a metal salt of diformylamide of formula (VIII) 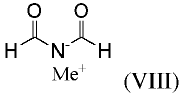 wherein Me⁺ is preferably sodium or potassium, followed by acid hydrolysis. In a fifth aspect, the invention provides for a process for making linezolid, wherein the benzoate salt of formula (II-D) reacts with an acetylation agent to form linezolid of formula (I).

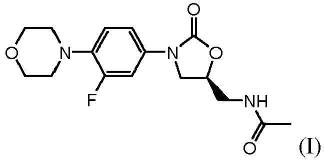

### DETAILED DESCRIPTION OF THE INVENTION

In each of the known processes for making linezolid of formula (I) 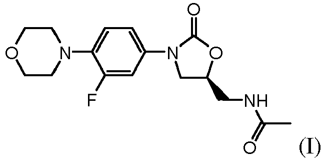 by acetylation of the amine-intermediate of formula (II), 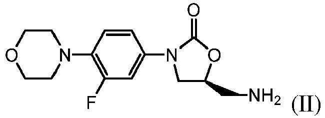 said amine intermediate must be provided as an (S)- enantiomer, because the acetylation process itself neither contributes to any improvement in configuration on the chiral carbon nor allows for removal of rests of the second enatiomer eventually present. Thus, the actual material comprising the compound (II) must exhibit high enantiomeric purity because further purification in this respect is either impossible or economically disadvantageous. High enantiomeric purity is of utmost importance particularly because of the high therapeutical dose of linezolid in pharmaceutical applications (generally up to 600 mg per day); accordingly, pharmaceutical regulations do not allow more than 0.1% of the undesired (R)-enantiomer of linezolid in the pharmaceutical product. Also, the chemical purity of the pharmaceutically acceptable linezolid must be very high.

The commercial process yielding linezolid is a multistep process. In the known process variants, a chiral carbon is formed in one of the early steps, and the same configuration of substituents on this carbon must be maintained throughout the subsequent steps. It is well known in the art that it is practically impossible to obtain a product with 100% enantiomeric purity (i.e. a product consisting solely of a single enantiomer) in a chemical process. On the other hand, there exist methods of improving the enantiomeric purity of a desired compound, the most common of which being a separation of a diastereomeric pair of salts or other derivatives with a chiral agent.

In accordance with the above, it is not surprising to a skilled person that the starting materials for making the compound of formula (II), particularly the alcohol of the formula (X), are sometimes obtainable only in 99% or less optical purity (i.e. comprising 1% or more of the undesired enantiomer of the title compound). Such quality is per se unsuitable for making linezolid of acceptable pharmaceutical quality and specific processes of enhancing the optical purity must be employed in proper time. However, no such purification process, e.g., a crystallization of a salt with a relatively expensive chiral acid, has been disclosed so far.

In the present invention, it was found with surprise that the compound of formula (II) may be advantageously provided and isolated in the form of a benzoate salt, wherein such salt may effectively solve the problem of removal of undesired (R)-enantiomer of formula (R)-(II) 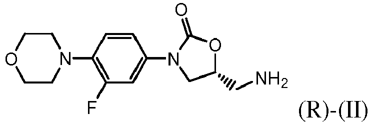 from mixtures comprising both enantiomers to a pharmaceutically acceptable level.

The term "benzoate salt" as used throughout the present specification is, in general, any benzoate salt compound that may be formed by contacting the compound of formula (II) with any molar equivalent amount of benzoic acid, and preferably is the monobenzoate compound of formula (II-D).

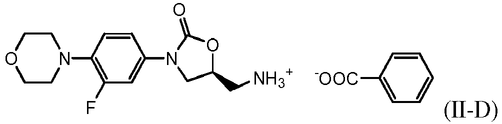

Providing and using the benzoate salt of the formula (II-D) is accompanied with several advantages:
a) The benzoate salt may be obtained as a nonhygroscopic crystalline material, which can be easily stored and handled. In long-term storage, the benzoate salt is more stable than the free base of the formula (II), particularly when exposed to aerial oxygen at elevated temperature and humidity;
b) Providing the benzoate salt in an isolated form from a solution in a suitable solvent is associated with a considerable purification effect, particularly in respect to enhancement of the optical purity of the compound (II);
c) In a process of providing the compound of formula (II), the step leading to a free base as an isolated species may be omitted;
d) The benzoate salt may be directly used in the subsequent acetylation reaction yielding linezolid of formula (I), without need of prior conversion into the free base.

The benzoate salt of the present invention is a salt comprising a cation derived from the N-(3-(3-fluoro-4-(morpholin-4-yl)phenyl)-2-oxooxazolidin-5(S)-ylmethyl)amine compound of formula (II) and an anion derived from benzoic acid. Accordingly, it may be produced by a salt-forming reaction between the compound of formula (II) and benzoic acid (or a salt thereof). As will be shown in more detail below, the compound of formula (II) may react in this salt-forming reaction "in situ", i.e. within a reaction mixture having provided the compound (II) by a chemical reaction.

While the above formula (II) and, accordingly, (II-D) correspond to a single (S) enantiomer of the N-(3-(3-fluoro-4-(morpholin-4-yl)phenyl)-2-oxooxazolidin-5-ylmethyl)amine (i.e. having 100% optical purity), it should be understood that the present invention also covers products comprising the same compound having optical purity lower than 100%, whenever relevant.

It is however preferred that the compound of the formula (II) is of an optical purity of 99.8% or higher, preferably 99.9% or higher. As the process of isolating the solid benzoate salt of formula (II-D) as discussed in detail below, particularly the precipitation thereof from alcoholic solvents, is accompanied with an improvement in optical purity, the compound of formula (II-D) of an optical purity of 99.8% or higher, preferably 99.9% or higher may be obtained accordingly, and such compound forms a particularly advantageous embodiment of the present invention.

In an advantageous mode of making the benzoate salt of the present invention, the compound of formula (II) reacts with benzoic acid in an organic solvent. The compound of formula (II) may be used in an isolated form, but a reaction mixture comprising it as a result of a chemical transformation may be used as well. Advantageously, the organic solvent is, alone or in admixture, an aliphatic, cyclic or aromatic hydrocarbon having from 5 to 12 carbon atoms, a halogenated hydrocarbon having from 1 to 6 carbon atoms, an aliphatic ester having from 2 to 10 carbon atoms, an aliphatic ketone having 3 to 8 carbon atoms, an aliphatic alcohol having from 1 to 5 carbon atoms, or mixtures thereof with water. In a yet advantageous mode, the formed benzoate salt is insoluble in the solvent and precipitates therefrom as a solid, typically crystalline, material, which may be isolated by common methods, for instance by filtration or centrifugation. For such purpose, an aliphatic alcohol having from 1 to 5 carbon atoms or mixtures thereof with water is the preferred solvent. The temperature of contacting the compound (II) with benzoic acid may range from ambient to reflux temperature. The temperature of precipitation and isolation of compound (II-D) is typically ambient temperature, but in some embodiments the reaction mixture with the precipitated solid may be cooled to a temperature below ambient, e.g. to a temperature of 10°C or less. Benzoic acid, which typically is charged into the reaction mixture as a solution in a suitable solvent, is advantageously used in a molar equivalent amount or in a slight (up to 1.2) molar excess.

The isolated product has typically the chemical structure corresponding to formula (II-D), i.e. it comprises both ions in a 1:1 molar ratio. The isolated solid product is a stable nonhygroscopic material (thermal analysis by DSC shows a single endotherm at about 179°C), which can be stored at ordinary conditions of storage and can be handled without special precautions. In an advantageous mode, it may be provided as a crystalline material. Such crystalline material typically comprises a distinctive XRPD pattern; a non-limiting example thereof is shown below. It is poorly soluble in water, contrary to any other known salt of the compound of formula (II), which allows using water in a process of isolation from a reaction mixture. In an important aspect, the product is also poorly soluble in an alcoholic solvent having from 1-5 carbon atoms, contrary to the free base (II) and to benzoic acid, which feature allows, in certain embodiments, easy making and pre-treatment of alcoholic solutions of both reaction partners as well as contacting them upon precipitation of the product without any particular adjustment of the temperature.

The unique solubility properties of the benzoate salt of compound (II) in water and alcohol solvents make the benzoate salt advantageous over other known salts of compound (II).

As an illustration, comparison of solubility of the benzoate and the acetate salts at 25°C (in g/100g of solvent) is given in the following table:

| | **Benzoate salt of (II)** | **Acetate salt of (II)** |
|---|---|---|
| Water | 1.83 | >78 |
| Methanol | 3.17 | 31.5 |
| Isopropanol | 0.3 | 0.95 |

The above process of precipitation of the benzoate salt of (II) from an organic solvent is associated with a considerable purification effect. Most surprisingly, even the optical purity of the compound (II) can be substantially improved by precipitation of the benzoate salt from a suitable solvent.

Thus, the present invention also provides for a process for improving the purity, particularly the optical purity, of the compound of formula (II), comprising contacting the compound of formula (II) with benzoic acid in a solvent, precipitating the benzoate salt of the formula (II-D), and isolating the solid from the reaction mixture. The solvent advantageously comprises an alcoholic solvent having from 1-5 carbon atoms, preferably methanol or isopropanol, and most advantageously it comprises an admixture of such alcoholic solvent with water, preferably with 0.5 - 10% , most preferably 2 to 7% of water.

The temperature of contacting the compound (II) with benzoic acid is preferably an ambient or higher than ambient (up to reflux) temperature. The temperature of the reaction mixture before isolation of the compound (II-D) is preferably an ambient or lower than ambient (up to 0°C) temperature. Isolation is carried out by conventional means, e.g. by filtration or centrifugation, and the obtained product may be optionally washed, dried or milled by conventional processes.

Typically, crude free base of formula (II) having a chemical purity of about 95% is purified by the above process to a chemical purity level of 98% or higher in a single crystallization. Such effect is considerably higher than that when converting the free base of (II) into a hydrochloride salt of the prior art, as shown in the following table comparing the chemical purity of the hydrochloride and benzoate salt, obtained from the same free base (II) in isopropanol:

| | |
|---|---|
| Free base | 94.15% |
| Hydrochloride | 95.91% |
| Benzoate | 99.83% |

Furthermore, the free base having an optical purity of from between 97 to 99% is purified to an optical purity level of 99.8% or higher when using conversion into the benzoate salt in accordance with the present invention. No such purification effect was observed when using the hydrochloride or acetate salt. This is illustrated by the data in the following table comparing the optical purity of a benzoate and a hydrochloride salt, made from the same free base (II) in isopropanol:

| | |
|---|---|
| Free base | 99.05% |
| Hydrochloride | 99.10% |
| Benzoate | 99.91% |

This level of purification is comparable with that obtainable by using salts of the compound (II) with optically active acids, e.g. with camphorsulfonic acid. The process of the present invention is however substantially cheaper as it does not require a relatively expensive chiral acid.

Thus, the benzoate salt (II-D) having a chemical purity of 99% or higher and/or having an optical purity of 99.8% or higher forms a specific aspect of the invention.

Optionally, the crystallized benzoate salt is converted back to the free base of formula (II) by neutralization with a strong, preferably inorganic, base in a solvent; after such neutralization, the free base of formula (II) with enhanced purity is isolated, e.g., by an extraction into a low polar water immiscible solvent, such as dichloromethane or ethyl acetate.

Alternatively, the benzoate salt of the present invention is used in the next reaction step leading to linezolid as shown below.

The starting compound of formula (II) for making the benzoate salt of the present invention can be made by any process known in the prior art. In particular, as shown above, it may be produced from a compound of formula (VII) 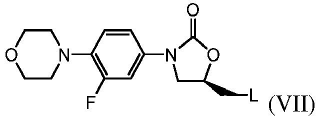 wherein L is a leaving group, typically a halo group or an alkyl- or aryl-sulfonyloxy group. The "halo" group comprises chloro- or bromo-group, preferably chloro- group. The "alkyl" comprises a C1-C4 alkyl group, preferably a methyl group. The "aryl" comprises a phenyl group, which may be optionally substituted by at least one C1-C4 alkyl group, a nitro-group, a hydroxyl group, a C1-C4 alkoxy group, and is preferably a p-tolyl or p-nitrophenyl group.

Accordingly, the benzoate salt of the present invention can be obtained by a process, characterised in that the leaving group L in the precursor of the formula (VII), wherein L is a halogen or an alkyl-or aryl sulfonyloxy group, is converted into an amino-group, whenever such process leading to the compound (II) is carried out in the presence of benzoic acid or a salt thereof, or whenever the compound (II) produced by conversion of compound (VII) is treated with benzoic acid or a salt thereof.

Methods of converting the leaving group L in the compound (VII) into the amino-group of the compound (II) are known in the art and a brief overview thereof is given above.

Advantageously, the compound of formula (II) is prepared by a process in which the compound of formula (VII) reacts, in an inert solvent, with a metal salt of diformylamide of formula (VIII).

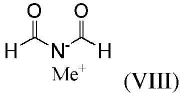

The "Me⁺" is typically sodium or potassium, preferably sodium. The reaction product comprises a mixture of compounds of formula (IXa) and (IXb).

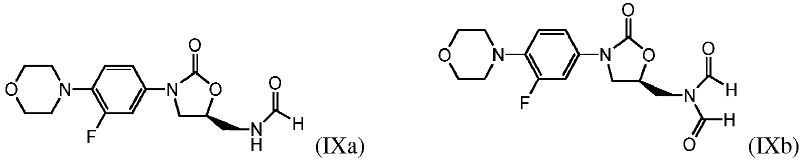

Such mixture is converted, in a next step, into the compound of formula (II) by hydrolysis of the formic group(s) by strong mineral or organic acid, for instance hydrochloric acid, sulphuric acid, phosphoric acid, or hydrobromic acid. The product of the hydrolytic reaction comprises the corresponding acid addition salt of the amine (II), which may be optionally neutralized into free amine by treatment with an equivalent of a base, optionally followed by an extraction, preferably into a low polar water immiscible organic solvent. The process details have been disclosed in WO 2012/019862. The solution of the free base of formula (II) so obtained may be directly used for making the benzoate salt of the present invention. Advantageously, such solution is mixed with a solution of benzoic acid in an alcohol, eventually heated to complete dissolution and the benzoate salt is allowed to precipitate from such solution. The precipitation may be carried out by cooling, seeding, partial evaporation of the solvent, addition of an antisolvent or a combination of these techniques.

The benzoate salt of the formula (II-D) may be used as an intermediate in making linezolid of formula (I). In an important and very advantageous aspect, the benzoate salt of the present invention may be directly converted to linezolid by reaction with an acetylation agent, which typically is an acetyl halide or acetic acid anhydride. Thus, there is no prior need to make the free base of the compound (II) from the salt.

In an advantageous way, the benzoate salt of the present invention is dissolved or suspended in an inert solvent, typically a low polar solvent such as a hydrocarbon or chlorinated hydrocarbon, and the acetylation agent, preferably acetic anhydride, is added to the stirred mixture. In a typical embodiment, the reaction is carried out at ambient temperature. The course of reaction may be monitored by a suitable method. After germination of the reaction, the formed benzoic and acetic acids are removed by neutralization and aqueous washing or extraction. Linezolid may be isolated from the solution comprising it by conventional ways, e.g. by evaporation of the solvent.

The produced linezolid may be isolated in, or subsequently converted to, any solid state form known in the art [ Form I (J.Med.Chem. 39(3), 673 (1996)), Form II (WO 01/057035, US 6,559,305), Form III (WO 2005/035530) and many others (WO 2006/004922 , US 2006/0142283), amorphous form (WO 2007/026369), and hydrated forms (US 2006/111350, EP 20033960 )] and/or may be converted to a suitable acid addition salt, e.g. to a hydrochloride.

The linezolid prepared by the process of the present invention can be formulated and used in pharmaceutical compositions. A suitable pharmaceutical composition may comprise linezolid and at least one pharmaceutically acceptable excipient. The compositions are useful as antibacterial agents, in treating various diseases caused by some types of bacteria, by administering an effective amount thereof to a patient in need of such treatment. In particular, they are useful in treatment of diabetic food infections caused by Gram-positive bacteria. Typically, the effective amounts range from 1 mg to 600 mg, expressed as the amount of linezolid base, per day.

The invention will be further described with reference to the following non-limiting examples.

### Examples

### Example 1

### 3-(3-fluoro-4-(morpholin-4-yl)phenyl)-2-oxooxazolidin-5(S)-ylmethyl)amine (II)

A 250 ml, three-necked, round-bottomed flask equipped with a blade mechanical stirrer and Ar inlet combined with a temperature probe was placed under an argon atmosphere and charged with sodium diformylamide (5.6 g) and the compound (VII) [ L= p-toluenesulfonyloxy group] (20.8 g). N,N-Dimethylformamide (52 ml) was added and the reaction mixture was heated to 85°C for 2 h. Hydrochloric acid (98 ml) was carefully added into the reaction mixture, such that the internal temperature did not exceed 96°C. The heterogeneous reaction mixture was heated to 85°C and the reaction was continued for 2.5 h. The reaction mixture was transferred to a 500 ml round-bottom flask and cooled in an ice-water bath. Dichloromethane (150 ml) was charged to the flask. The mixture was vigorously stirred and a solution of NaOH (54.0 g) in water (100 ml) was added, such that the internal temperature did not exceed 20°C. The mixture was filtered. The filtrate was transferred to a 500 ml separatory funnel and the organic layer was separated. The filter cake was discarded. The water layer was extracted with dichloromethane (2 x 50 ml). The combined organic layer was dried over Na2SO4 (25 g), filtered, and concentrated (60°C, 50 mbar) to provide the product as a tan crystalline solid (18.87 g).

### Example 2

### 3-(3-fluoro-4-(moxpholin-4-yl)phenyl)-2-oxooxazolidin-5(S)-ylmethyl)amine benzoate (II-D)

5.00 g of the free base of the compound (II) (0.0169 mol) from Example 1 was dissolved in a mixture of 75.0 ml of isopropanol and 1.50 ml of demi water (2% v/v on i-PrOH) at 80°C. Then 2.064 g of benzoic acid (0.169 mol) in 4.13 ml of methanol was added dropwise in 30 min. The suspension was stirred at 80°C for 1 h. Then it was cooled to RT (1 h) and stirred at RT for 1 h. Product was filtered, washed with 2x 10 ml of isopropanol and dried at RT in vacuo at 50°C overnight.
Yield: 6.315 g (89.5% of theor. yield) white crystals
Structure conforms with the formula (II-D) [NMR]
DSC (10°C/min): single endotherm 179.08 °C

### Example 3

### Purification of compound (II)

3.240 g (10.97 mmol) of compound (II) having a chemical purity of 94.15% (HPLC) and an optical purity of 99.05% (HPLC) was dissolved in 50 ml of a mixture of isopropanol and water (95:5 v/v) upon stirring and heating to 81°C (ext.,reflux) and 1.340 g (1.00 equiv.) of benzoic acid in 3.00 ml of methanol was added (clear solution). The solution was spontaneously cooled to RT and stirred at RT for 1 h. The solid product was filtered and washed with 3x 5.00 ml of isopropanol and dried at RT in vacuo.
Yield: 3.811 g (83.0% of theor. yield) of white crystals
Chemical purity: 99.83% (HPLC)
Optical purity: 99.91% (HPLC)
XRPD (characteristic peaks, ± 0.2 degrees 2 theta):

| Angle( 2 theta degrees) | d value (Angstrom) | Intensity ( count% ) |
|---|---|---|
| 19.998 | 4.43864 | 100 |
| 26.666 | 3.34024 | 62.4 |
| 3.743 | 23.58529 | 43 |
| 26.924 | 3.3089 | 34.9 |
| 27.719 | 3.21568 | 26.1 |
| 21.29 | 4.17001 | 25.1 |
| 21.69 | 4.09398 | 21.5 |
| 22.027 | 4.03209 | 20.4 |
| 14.373 | 6.15762 | 19.9 |
| 28.303 | 3.15068 | 18.3 |
| 27.258 | 3.26903 | 18.1 |
| 20.691 | 4.28946 | 18.0 |
| 15.214 | 5.81912 | 15.3 |
| 22.904 | 3.87967 | 12.4 |
| 13.356 | 6.62383 | 12.1 |
| 19.104 | 4.64193 | 11.1 |
| 28.992 | 3.07736 | 10.7 |
| 16.366 | 5.41172 | 8.9 |
| 20.288 | 4.37369 | 8.6 |
| 16.171 | 5.47661 | 8.4 |
| 24.293 | 3.66091 | 8.3 |
| 24.48 | 3.63335 | 8.2 |
| 9.975 | 8.86017 | 6.7 |
| 26.245 | 3.39292 | 6.5 |
| 30.292 | 2.94823 | 6.4 |
| 24.866 | 3.57786 | 6.4 |

The XRPD spectrum was recorded according to the following settings:
Start angle (2θ): 2.0°
End angle (2θ): 35.0°
Scan step width: 0.02°
Scan step time: 1.5 sec.
Radiation type: Cu
Radiation wavelengths: 1.54060 Å (Kα₁), primary monochromator used
Exit slit: 6.0 mm
Focus slit: 0.2 mm
Divergence slit: Variable (V20)
Antiscatter slit: 11.8 mm
Receiving slit: 20.7 mm

### Example 4

### Conversion of compound (II-D) to linezolid

3.600 g of the benzoate salt (II-D) was suspended in 30.00 g of dichloromethane and the temperature was set up to 24°C (thick suspension). Then 1.145 g of acetanhydride was dropped in the course of 15 min. (clear solution). The mixture was stirred at RT. HPLC sample after 60 minutes confirmed complete conversion.

The solution was neutralized with a solution of 0.92 g of sodium hydroxide in 4.40 ml of deionized water at 20 - 30°C and the mixture was diluted with 2.60 ml of water. The layers were separated, the organic one was washed with 2x 7.0 ml of water. The organic solution was filtered with 100 mg of charcoal and the cake was washed with 3.4 ml of dichloromethane. The filtrate was evaporated (70°C, 100 mbar).
Yield: 2.825 g (97% of theoretical yield) of white crystals of linezolid.

## Claims

1. A benzoate salt of formula (II-D)

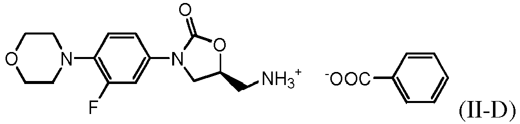

2. The salt according to claim 1 in a solid form.

3. The salt according to claim 2 in a crystalline form.

4. The salt according to any one of claims 1-3 having an optical purity of 99.8% or higher and/or a chemical purity of 99% or higher.

5. A process for making the benzoate salt of formula (II-D) according to any one of claims 1-4, comprising reacting the compound of formula (II) 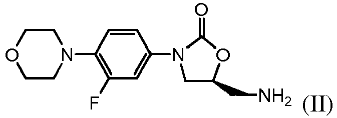 with benzoic acid in a solvent.

6. The process according to claim 5, wherein the solvent is, alone or in admixture, an aliphatic, cyclic or aromatic hydrocarbon having from 5 to 12 carbon atoms, a halogenated hydrocarbon having from 1 to 6 carbon atoms, an aliphatic ester having from 2 to 10 carbon atoms, an aliphatic ketone having 3 to 8 carbon atoms, an aliphatic alcohol having from 1 to 5 carbon atoms, or mixtures thereof with water, preferably an aliphatic alcohol having from 1 to 5 carbon atoms, or mixtures thereof with water.

7. The process according to claim 5 or 6, wherein the benzoate salt is isolated from the reaction mixture in a solid, preferably crystalline, form.

8. A process for making the benzoate salt of formula (II-D) according to any one of claims 1-4, **characterised in that** the leaving group L in the precursor of formula (VII) 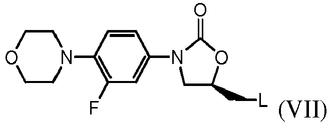 wherein L is a halo- group or an alkyl- or aryl sulfonyloxy group,
is converted into an amino-group in the presence of, or followed by a treatment with, benzoic acid.

9. The process according to claim 8, wherein the phenyl group of the aryl sulfonyloxy group is substituted by at least one C1-C4 alkyl group, a nitro group, a hydroxyl group or a C1-C4 alkoxy group.

10. The process according to claim 8 or 9, wherein the group L is converted into an amino-group by reaction of the compound of formula (VII) with a metal salt of diformylamide of formula (VIII) 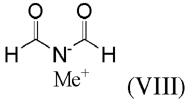 wherein Me is preferably sodium or potassium,
followed by acid hydrolysis.

11. A process for improving the purity, particularly the optical purity, of the compound of formula (II) comprising converting the compound of formula (II) into the benzoate salt of formula (II-D) in a solvent, precipitating and isolating the benzoate salt and converting the isolated benzoate salt into the compound of formula (II).

12. The process according to claim 11, wherein the solvent comprises an alcoholic solvent of 1-5 carbon atoms, preferably methanol or isopropanol.

13. The process according to claim 12, wherein the solvent comprises an admixture of the alcoholic solvent with water, preferably with 0.5 - 10% of water.

14. A process for making linezolid, wherein the benzoate salt of formula (II-D) reacts with an acetylation agent to form linezolid of formula (I).

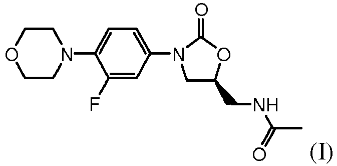

15. The process according to claim 14, wherein the reaction proceeds in an organic solvent.

16. Use of the benzoate salt of formula (II-D) in a process for purification of the compound of formula (II) according to any one of claims 11-13 or for making linezolid of formula (I) according to claim 14 or 15.

## Patentansprüche

1. Benzoatsalz der Formel (II-D)

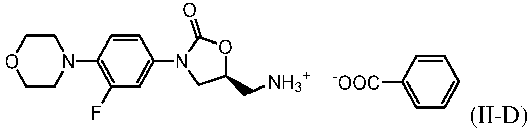

2. Salz nach Anspruch 1 in einer festen Form.

3. Salz nach Anspruch 2 in einer kristallinen Form.

4. Salz nach einem beliebigen der Ansprüche 1-3 mit einer optischen Reinheit von 99,8% oder höher und/oder einer chemischen Reinheit von 99% oder höher.

5. Verfahren zum Herstellen des Benzoatsalzes der Formel (II-D) gemäß einem
beliebigen der Ansprüche 1-4, umfassend Reagieren der Verbindung der Formel (II) 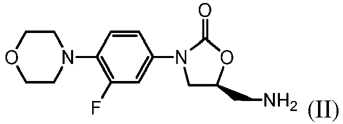 mit Benzoesäure in einem Lösungsmittel.

6. Verfahren nach Anspruch 5, wobei das Lösungsmittel, alleine oder in Mischung, ein aliphatischer, cyclischer oder aromatischer Kohlenwasserstoff mit von 5 bis 12 Kohlenstoffatomen, ein halogenierter Kohlenwasserstoff mit von 1 bis 6 Kohlenstoffatomen, ein aliphatischer Ester mit von 2 bis 10 Kohlenstoffatomen, ein aliphatisches Keton mit 3 bis 8 Kohlenstoffatomen, ein aliphatischer Alkohol mit von 1 bis 5 Kohlenstoffatomen, oder Gemische davon mit Wasser ist, bevorzugt ein aliphatischer Alkohol mit von 1 bis 5 Kohlenstoffatomen, oder Gemische davon mit Wasser.

7. Verfahren nach Anspruch 5 oder 6, wobei das Benzoatsalz aus dem Reaktionsgemisch in einer festen, bevorzugt kristallinen, Form isoliert wird.

8. Verfahren zum Herstellen des Benzoatsalzes der Formel (II-D) gemäß einem beliebigen der Ansprüche 1-4, **dadurch gekennzeichnet, dass** die Abgangsgruppe L im Präkursor der Formel (VII) 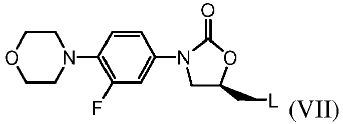 wobei L eine Halogruppe oder eine Alkyl- oder Arylsulfonyloxygruppe ist,
in der Gegenwart von, oder gefolgt durch eine Behandlung mit, Benzoesäure in eine Aminogruppe umgewandelt wird.

9. Verfahren nach Anspruch 8, wobei die Phenylgruppe der Arylsulfonyloxygruppe durch mindestens eine C1-C4-Alkylgruppe, eine Nitrogruppe, eine Hydroxylgruppe oder eine C1-C4-Alkoxygruppe substituiert ist.

10. Verfahren nach Anspruch 8 oder 9, wobei die Gruppe L in eine Aminogruppe umgewandelt wird durch Reaktion der Verbindung der Formel (VII) mit einem Metallsalz von Diformylamid der Formel (VIII) 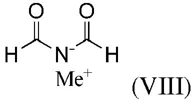 wobei Me bevorzugt Natrium oder Kalium ist,
gefolgt von Säurehydrolyse.

11. Verfahren zum Verbessern der Reinheit, insbesondere der optischen Reinheit, der Verbindung der Formel (II), umfassend Umwandeln der Verbindung der Formel (II) in das Benzoatsalz der Formel (II-D) in einem Lösungsmittel, Präzipitieren und Isolieren des Benzoatsalzes, und Umwandeln des isolierten Benzoatsalzes in die Verbindung der Formel (II).

12. Verfahren nach Anspruch 11, wobei das Lösungsmittel ein alkoholisches Lösungsmittel von 1-5 Kohlenstoffatomen umfasst, bevorzugt Methanol oder Isopropanol.

13. Verfahren nach Anspruch 12, wobei das Lösungsmittel eine Mischung des alkoholischen Lösungsmittels mit Wasser, bevorzugt 0,5 - 10% Wasser umfasst.

14. Verfahren zum Herstellen von Linezolid, wobei das Benzoatsalz der Formel (II-D) mit einem Acetylierungsmittel reagiert, um Linezolid der Formel (I) zu bilden.

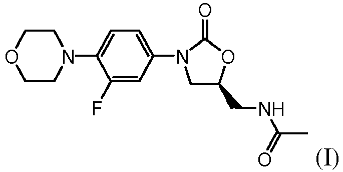

15. Verfahren nach Anspruch 14, wobei die Reaktion in einem organischen Lösungsmittel vonstattengeht.

16. Verwendung des Benzoatsalzes der Formel (II-D) in einem Verfahren zur Reinigung der Verbindung der Formel (II) gemäß einem beliebigen der Ansprüche 11-13 oder zum Herstellen von Linezolid der Formel (I) gemäß Anspruch 14 oder 15.

## Revendications

1. Un sel de benzoate de formule (II-D)

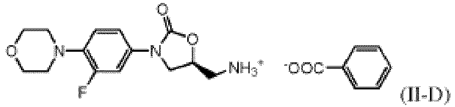

2. Le sel selon la revendication 1, sous forme solide.

3. Le sel selon la revendication 2, sous forme cristallisée.

4. Le sel selon une quelconque des revendications 1 à 3, présentant une pureté optique d'au moins 99,8% et/ou une pureté chimique d'au moins 99%.

5. Un procédé pour fabriquer le sel de benzoate de formule (II-D) selon une quelconque des revendications 1 à 4, comprenant la réaction du composé de formule (II) 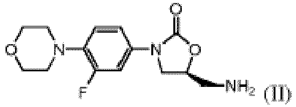 avec de l'acide benzoïque dans un solvant.

6. Le procédé selon la revendication 5, dans lequel le solvant, utilisé seul ou en mélange, est un hydrocarbure aliphatique, cyclique ou aromatique comportant de 5 à 12 atomes de carbone, un hydrocarbure halogéné comprenant de 1 à 6 atomes de carbone, un ester aliphatique comprenant de 2 à 10 atomes de carbone, une cétone aliphatique comprenant de 3 à 8 atomes de carbone, un alcool aliphatique comprenant de 1 à 5 atomes de carbone ou des mélanges de ceux-ci, avec de l'eau, de préférence un alcool aliphatique comprenant de 1 à 5 atomes de carbone ou des mélanges de celui-ci avec de l'eau.

7. Le procédé selon la revendication 5 ou 6, dans lequel le sel de benzoate est isolé du mélange réactionnel sous forme solide, de préférence sous forme cristallisée.

8. Un procédé de fabrication de sel de benzoate de formule (II-D) selon une quelconque des revendications 1 à 4, **caractérisé en ce que** le groupe partant L du précurseur de formule (VII) 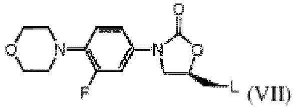 formule dans laquelle L est un groupe halo- ou un groupe alkyl- ou un groupe aryl sulfonyloxy
est converti en un groupe amino en présence d'acide benzoïque, ou suite à un un traitement avec l'acide benzoïque.

9. Le procédé selon la revendication 8, dans lequel le groupe phényle du groupe aryl-sulfonyloxy est substitué par au moins un groupe (C₁-C₄)-alkyle, un groupe nitro, un groupe hydroxyl ou un groupe (C₁-C₄)-alkoxy.

10. Le procédé selon la revendication 8 ou 9, dans lequel le groupe L est converti en un groupe amino par réaction du composé de formule (VII) avec un sel métallique de diformylamide de formule (VIII) 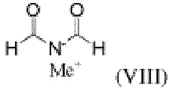 formule dans laquelle Me est de préférence du sodium ou du potassium, réaction que l'on fait suivre d'une hydrolyse dans des conditions acides.

11. Un procédé pour améliorer la pureté, particulièrement la pureté optique du composé de formule (II) comprenant la conversion dans un solvant du composé de formule (II) en le sel de benzoate de formule (II-D), la précipitation et l'isolement du sel de benzoate puis la conversion du sel de benzoate isolé en le composé de formule (II).

12. Le procédé selon la revendication 11, dans lequel le solvant comprend un solvant alcoolique comportant 1 à 5 atomes de carbone, de préférence du méthanol ou de l'isopropanol.

13. Le procédé selon la revendication 12, dans lequel le solvant comprend un mélange de solvant alcoolique et d'eau, de préférence de 0,5 à 10% d'eau.

14. Un procédé de fabrication de linézolide, dans lequel le sel de benzoate de formule (II-D) est amené à réagir avec un agent d'acétylation pour former le linézolide de formule (I).

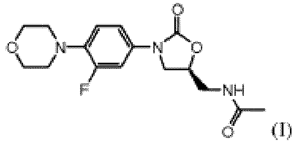

15. Le procédé selon la revendication 14, dans lequel la réaction se déroule dans un solvant organique.

16. Utilisation du sel de benzoate de formule (II-D) dans un procédé pour la purification du composé de formule (II) selon une quelconque des revendications 11 à 13 ou pour fabriquer le linézolide de formule (I) selon la revendication 14 ou 15.
